Europäisches Patentamt

European Patent Office

Office européen des brevets

(19)

(11) Publication number: **0 335 854**
A2

(12) **EUROPEAN PATENT APPLICATION**

(21) Application number: 89870044.8

(22) Date of filing: 29.03.89

(51) Int. Cl.⁴: **C 12 N 15/00**
C 12 P 21/02, C 12 R 1/38,
C 12 R 1/425, C 12 R 1/18

(30) Priority: 30.03.88 US 175082

(43) Date of publication of application:
04.10.89 Bulletin 89/40

(84) Designated Contracting States:
AT BE CH DE ES FR GB GR IT LI LU NL SE

(71) Applicant: MONSANTO COMPANY
800 North Lindbergh Boulevard
St. Louis, MO 63167 (US)

(72) Inventor: **Drahos, David Joseph**
13320 Hobnail Drive
St. Louis Missouri 63146 (US)

**Olins, Peter Olafs**
16464 Birch Forest Drive
Ballwin Missouri 63011 (US)

**Fuchs, Roy Lee**
917 Weatherstone Drive
St. Charles Missouri 63303 (US)

**Rangwala, Shaukat Husaini**
925 Cleta Drive
Ballwin Missouri 63021 (US)

(74) Representative: **Lunt, John Cooper et al**
Monsanto Europe S.A. Patent Department Avenue de
Tervuren 270-272 Letter Box No 1
B-1150 Brussels (BE)

The microorganism(s) has (have) been deposited with American Type Culture Collection under numbers ATCC 39936 - ATCC 53469 - ATCC 67043 - ATCC 67044 - ATCC 53023.

(54) **Regulated gene expression in gram-negative microorganisms.**

(57) The present invention provides methods and compositions useful in causing inducible expression of desired proteins in non-*E. coli* (e.g. heterologous) Gram-negative microorganisms.

EP 0 335 854 A2

## Description

## Regulated Gene Expression in Gram-Negative Microorganisms

TECHNICAL FIELD

The present invention relates to a method and means for regulating expression of structural genes encoding desired polypeptides in Gram-negative microorgamisms.

BACKGROUND OF THE INVENTION

The structure of a number of promoters from *E. coli* and from *E. coli* bacteriophages has been established and their function extensively investigated. Knowledge pertaining to these promoters has since enabled the production of numerous medically and agriculturally important substances in *E. coli* by conventional recombinant DNA techniques. For other Gram-negative bacteria, however, little information is available on the structure and function of their respective promoters thereby impeding the use of such bacteria as host cells for recombinant DNA production of commercially and economically valuable substances.

The development of alternative Gram-negative host cells for production of medically and agriculturally important substances is advantageous for several reasons. The employment of Gram-negative bacteria other then *E. coli* would avoid the concern over possible endotoxin contamination in products produced in *E. coli* host cells and avoid the necessity of developing or isolating *E. coli* strains devoid of human endotoxins. Furthermore, the use of such Gram-negative bacteria as *Pseudomonas* as a host cell would provide great economic savings owing to both the metabolic and physiologic properties of *Pseudomonas*. Under certain conditions, *Pseudomonas*, for example, can be grown to higher cell culture densities than *E. coli* thus providing potentially greater product yields. Additionally, *Pseudomonas* can be grown on a greater number of nutrient sources thus providing greater production flexibility and possible cost savings. Similarly, Gram-negative microorganisms such as *Pseudomonas,Serratia* and *Erwinia* actively excrete a number of proteins into the growth medium. It is desirable to develop expression systems which can harness such excretion pathways to secrete or excrete desired peptides or proteins. Also, *Pseudomonas* can also be cultured in growth media devoid of antibiotics owing to its production of siderophores, iron chelating molecules, which effectively starves potentially contaminating microorganisms of vital metabolic iron molecules.

There is, therefore, a need to develop economically attractive expression systems for Gram-negative bacteria in addition to *E. coli*. Ideally, these expression systems should be of the type that afford on-off (e.g. regulatable or inducible) control of desired protein production in these new host cells so that such systems can be used commercially.

Production, in any cell, of a protein from a selected DNA sequence encoding the protein, first requires that the DNA sequence be transcribed into messenger RNA (mRNA) and, second, that the mRNA then be translated into the protein. Transcription is initiated by the binding of an enzyme, RNA polymerase, to a promoter sequence in the DNA. Thus, the promoter sequence must be one that is recognized by the host cell RNA polymerase. RNA polymerase then catalyzes the conversion of DNA into a mRNA molecule. The subsequent translation of the mRNA molecule into protein is initiated by the binding of the mRNA molecule to cellular ribosomes. The mRNA molecule must, therefore, contain a binding site recognized by the host cell ribosomes.

Absent an understanding and identification of non-*E. coli* promoters and ribosome binding sites for use in the recombinant DNA production of desired substances (e.g. proteins or polypeptides), investigators have begun exploring the possibility of using *E. coli* promoters and ribosome binding sites in other Gram-negative bacteria.

Studies of the interchangeability of *E. coli* and *Pseudomonas* promoters indicate that while some *E. coli* promoters can function in *Pseudomonas*, most in a non-regulatable fashion, the reverse is not always the case. The lack of such interchangeability is not surprising as there is little evidence of significant exchange of chromosomal DNA between Gram-negative microorganisms and, hence, only a slight opportunity for such organisms to adapt their respective enzymes and/or expression machinery to the recognition of regulatory signals of other organisms. Indeed, recent studies of known *Pseudomonas* promoter structure indicate significant variation from *E. coli* consensus promoter structure. See Jeenes, D. J. et al. Mol. Gen. Genet. (1986) 203:421-429. Such studies indicate a lack of a currently definable mechanism by which some *E. coli* promoters initiate transcription in *Pseudomonas* as well as supporting the unpredictability of *E. coli* promoter function in *Pseudomonas*.

Regulation, for example, of the on-off control of promoter-initiated transcription requires interactions between inducing (e.g. on-regulating) substances and promoters. The nature of these interactions is not well understood especially in non-*E. coli* Gram-negative bacteria. Typically, the inducing substance(s) interacts specifically with repressor molecules within the cell in such a manner as to inhibit the repressor's ability to bind the promoter region. The inhibition of repressor binding causes the activation of transcription. Thus, in order for a promoter from one genus of organism to function in a regulatable fashion in a second genus of organism, the second organism should have a repressor(s) that interacts with the inducing substance in such a precise manner as to inhibit the repressor from binding to the promoter region. Controlled on-off regulation of a gene, therefore, involves complex and specific interactions within the cell beyond the mere recognition of a promoter region by a host cell RNA polymerase.

It is believed that most Gram-negative organisms have a protein or proteins that are involved in the repair of damaged DNA. In *E. coli*, one such protein is the *rec*A protein. Benbrook and Miller (Antimicrob. Agents Chemother., (1986), 29:1-6), however, have shown that while *Pseudomonas aeurginosa* contains a genetic analogue of the *E. coli rec*A gene, *Pseudomonas rec*A gene expression is not induced by such substances as naladixic acid or norfloxicin which do induce *E. coli rec*A protein production. Thus, while a genetic analogue of the *E. coli rec*A gene exists in *Pseudomonas*, the control (e.g. regulation) of the *Pseudomonas rec*A protein differs from *E. coli*. One would, therefore, not expect an *E. coli rec*A promoter to act in a regulatable fashion in *Pseudomonas* and certainly not under the same regulation applied in *E. coli*.

It is also clear that the regulation of known *E. coli* promoters and efficiency of known *E. coli* ribosomes binding sites in other Gram-negative bacteria has not been established. It is, therefore, desirable to develop an effective, efficient, tightly controlled (e.g. regulatable) and economically attractive expression system operable in a variety of Gram-negative bacteria.

It is an objective of the present invention to provide a regulatable expression system operable in Gram-negative bacteria, in addition to *E. coli*.


SUMMARY OF THE INVENTION


In one embodiment, the present invention provides a method for causing regulatable expression of a structural gene encoding a desired polypeptide in heterologous Gram-negative bacteria, said method comprises inducing an *E. coli rec*A promoter operatively linked to the structural gene and thereby obtaining the polypeptide. In a preferred embodiment, the *E. coli rec* A promoter and structural gene are also operatively joined to a bacteriophage T7 ribosome binding site which effects enhanced production of desired protein.

In a further embodiment, the present invention provides recombinant DNA expression vehicles comprising a broad host range origin of replication and an *E. coli rec*A promoter operatively linked to a structural gene encoding a desired polypeptide wherein the *rec*A promoter is capable of directing inducible expression in a heterologous Gram-negative bacteria.

Also provided are recombinant non-*E. coli* Gram-negative bacteria, the genomes of which comprise an *E. coli rec*A promoter, capable of employing the expression vehicles and methods of the present invention to produce desired polypeptides.


BRIEF DESCRIPTION OF THE FIGURES


In the following diagrammatic representations, the nucleic acid sequences are provided in a 5′ to 3′ orientation unless otherwise noted and wherein the nucleosides adenosine, guanine, cytosine and thymine are denoted by A, G, C and T, respectively. The directional arrows represent the 5′ to 3′ orientation of the DNA coding sequences. "prec" denotes the *E. coli rec*A promoter, "P$_L$" denotes the bacteriophage lambda P$_L$ promoter, "SD" denotes a SD-sequence, "amp$^r$" denotes an ampicillin resistance gene, "Gm$^r$" denotes a gentamicin resistance gene, "LacZ" denotes the beta-galactosidase structural gene, "kb" denotes kilobases and "ori" denotes the origin of replication. Relevant restriction endonuclease sites are also shown. The DNA regions so marked are for diagrammatic purposes only and are not drawn to scale unless otherwise noted.

Figure 1 depicts the DNA sequence of a synthetic double-stranded G10L sequence wherein the underlined nucleotides denote nucleotides which differ from the naturally occurring bacteriophage T7 gene 10 nucleotides and (†) denotes the SD-sequence. The (NdeI) denotes the location of the NdeI restriction endonuclease site in the naturally occurring bacteriophage T7 gene 10 sequence.

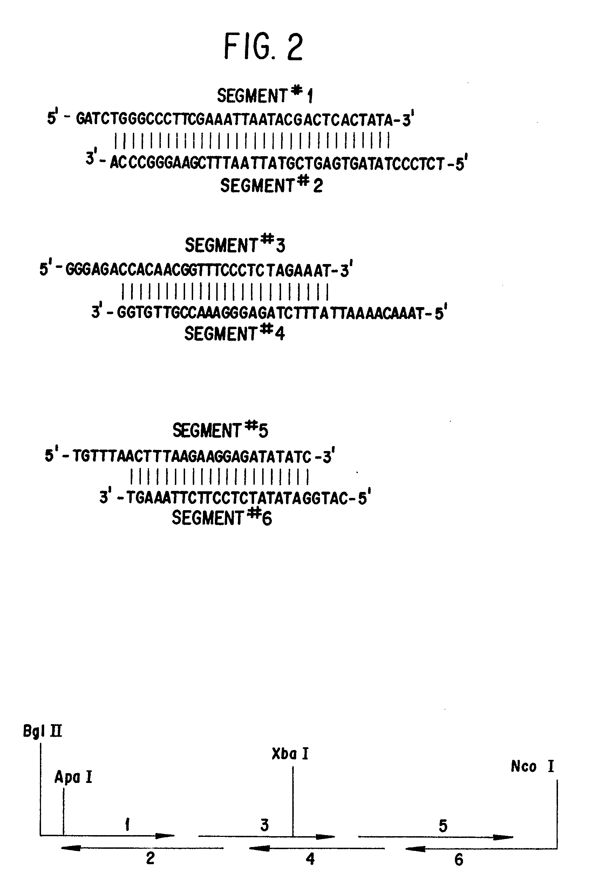

Figure 2 depicts the construction of the synthetic G10L sequence shown in Figure 1. Segments #1-#6 denote the individually synthesized oligonucleotides.

Figure 3 depicts the construction of pMON5515 comprising P*rec*, a G10L sequence (G10L) and an atriopeptigen (APgen) structural gene.

Figure 4 depicts the construction of pMON5542 comprising a pEMBL plasmid having inserted therein P*rec*, a G10L sequence and a *lac*Z structural gene (LacZ).

Figure 5 depicts the construction of two broad host range expression vehicles, pMON5757 and pMON5758, each comprising an IncQ replicon (IncQ), a gentamicin resistance selectable marker gene (Gm$^r$), P*rec*, a G10L sequence and a beta-galactosidase structural gene (LacZ).

Figure 6 depicts the relevant contents of plasmids pMON5014, pMON5756, pMON5759, pMON5760 and pMON5761.

DETAILED DESCRIPTION

The present invention relates to the discovery that the *E. coli recA* promoter is able to cause expression of desired structural gene in Gram-negative microorganisms other than *E. coli*. Additionally, the present invention relates to the further discovery that the *E. coli recA* promoter is inducible in heterologous Gram-negative microorganisms and can thus cause regulatable production of desired products in said microorganisms.

These discoveries now enable the construction of novel, recombinant heterologous (e.g. non-*E. coli*) Gram-negative bacteria, the genomes of which comprise an *E. coli recA* promoter, useful in producing desired products. These discoveries further enable the construction of recombinant expression vehicles capable of directing, in a preferred embodiment, the inducible expression of desired structural genes in a wide range of microorganisms and, hence, the use of said microorganisms for the commercial production of medically, chemically, and agriculturally important products.

1. The *E. coli recA* promoter

The *recA* gene of *E. coli* has been cloned and its nucleotide sequence determined (Horii et al., 1980, Proc. Nat'l. Acad. Sci., U.S.A. 77: 313-317; Sancar et al., 1980, Proc. Nat'l. Acad. Sci., U.S.A. 77: 2611-2615). Thus, the *E. coli recA* promoter/operator region, hereinafter referred to as "P*rec*", can be constructed by such conventional means as chemical synthesis and/or isolation from an appropriate genomic library. Additionally, P*rec* can be isolated from plasmid pMON6002, which is contained within *E. coli* JM101 having American Type Culture Collection (ATCC) accession number 67044, as a SalI-BglII restriction fragment.

As previously discussed, P*rec* controls the expression of the *E. coli recA* protein which functions generally as a DNA repair enzyme. Upon induction of the *E. coli recA* promoter, a series of repair enzymes are coordinately induced. (Kenyon, C.J. et al., 1982, J. Mol. Biol. 160: 445-457; Walker, G.D., 1984, Microbiol. Rev. 48: 60-93). Induction of P*rec* and coordinate genes is thus achieved, in *E. coli*, by conditions which damage or modify DNA. Such conditions include, without limitation, addition of such substances as nalidixic acid, norfloxicin, inhibitors of *E. coli* DNA gyrase or mitomycin C to the culture medium, ultraviolet radiation, temperature elevation and thymine starvation. As is described more fully herein, the present invention is directed to the discovery that the *E. coli recA* promoter can be similarly regulated in heterologous (i.e. non-*E. coli*) Gram-negative bacteria to cause inducible expression in said bacteria of desired structural genes. Thus, based on this discovery, it is understood that a compound(s) and/or condition(s) that induces P*rec* in *E. coli* would be able to induce P*rec* in heterologous Gram-negative bacteria. It is, furthermore, anticipated, based upon the discovery described herein, that the promoters controlling the expression of coordinately expressed DNA repair enzymes (Kenyon, C.J. et at., 1982; Walker, G.C., 1984) may alternatively be employed to cause inducible expression of desired structural genes in heterologous Gram-negative bacteria.

In one embodiment of the present invention, nalidixic acid acid is employed as the inducing agent or condition. The optimum concentration of nalidixic acid for purposes of inducing expression of DNA sequences under the control of the *E. coli recA* promoter can be determined by conventional techniques and may differ depending upon the host cell and medium chosen. A preferred concentration range of nalidixic acid is from about 50 to about 1000 µg/ml, with a most preferred concentration being from about 50 to 100 µg/ml. Optimization of other conditions, described above, which conditions are able to induce the *E. coli recA* promoter and coordinately expressed (i.e. induced) promoters, can be achieved by conventional means.

The operative joining of P*rec* to a DNA molecule comprising, for example, a structural gene and/or various transcription and translation regulatory sequences (e.g. expression components), can be achieved biologically and/or by enzymatic and/or chemical means including, for example, by means of a ligase. In the resultant recombinant DNA molecule, P*rec* and DNA coding sequences and/or components or other proteins associated with production of a desired peptide can be contiguous or noncontiguous, limited only by the ability of P*rec* to effect transcription of a desired structural gene. It is understood that P*rec* can be joined to a structural gene encoding a protein or enzyme that affects production or activity of a desired product.

In one embodiment, P*rec* is operatively joined to various procaryotic and eucaryotic structural genes contained within both limited and broad host range cloning and expression vectors. The operative joining of P*rec* to structural genes contained within broad host range expression vehicles led to the discovery that P*rec* can cause regulatable expression in a wide range of Gram-negative bacteria.

2. Preparation of Structural Genes Coding for Desired Polypeptides

The term "structural gene" is herein understood to mean a DNA sequence or molecule coding for a desired polypeptide or fragment thereof. Such DNA sequences or molecules can be chemically synthesized, isolated from appropriate genomic or cDNA libraries and/or enzymatically constructed by conventional means. Once chemically synthesized or isolated, the structural genes and/or other DNA sequences of the present invention, as described more fully hereinafter, represent essentially pure nucleic acid sequences or molecules. Thus, the term "essentially pure" when used to describe the nucleic acid (e.g. DNA or RNA) sequences or molecules of the present invention is understood to mean nucleic acid sequences free from nucleic acid sequences with which they are associated in a nature. Examples of such essentially pure nucleic acid sequences or molecules include, but are not limited to, nucleic acid sequences enzymatically or chemically released from a larger

4

(naturally occurring) molecule, chemically synthesized nucleic acid sequences free from naturally occurring intervening or contiguous sequences and nucleic acid sequences in combination with sequences or molecules with which they are not naturally found associated or combined. Thus, due to the way by which essentially pure molecules are created, they are sometimes alternatively referred to as "synthetic" nucleic acid sequences or molecules.

When the amino acid sequence of the desired polypeptide is known, a plurality of structural genes or DNA coding sequences can be constructed based upon the genetic code. Selection of the specific amino acid codons to be employed in constructing a structural gene is generally guided by such factors as determining a sequence which will optimize transcription and translation (e.g. expression of the structural gene) and accumulation of the desired gene product in the host cell chosen to produce the desired protein.

The products encoded in the structural genes or affected by proteins encoded in such genes are typically useful peptides or polypeptides. Such products can include proteins of both procaryotic and eucaryotic origin. While the developments described herein are demonstrated to yield the successful expression of structural genes encoding such animal growth factors as porcine growth hormone and such procaryotic enzymes as beta-galactosidase, it will be appreciated that the term "structural gene" includes DNA sequences encoding virtually any known protein or amino acid sequence. Such proteins include, but are not limited to, nerve growth factor, atrial peptide, insulin-like growth factor, transforming growth factor-alpha, epidermal growth factor, tissue plasminogen activator, viral antigens, interleukins and bovine growth hormone. Furthermore, polyamino acids, fusion proteins, regulatory proteins, proteases, polypeptides amenable to secretion or excretion and peptide fragments can also be encoded in a structural gene of the present invention and thereby produced in accordance with the present invention. It is understood that these and other proteins produced in recombinant microorganisms, in accordance with the materials and methods of the present invention, can be recovered and/or reconstituted to their respective native conformation by means known to those of skill in the art.

3. Expression Vehicles

An expression vector or vehicle is herein understood to comprise a DNA molecule such as a phage, plasmid, transposon or cosmid DNA capable of transforming a bacterial host cell and capable of causing expression of a desired gene in said host cell. For vectors that can actively maintain their presence in a recombinant host cell, such vectors typically contain an origin of replication (ori), genes encoding enzymes or proteins necessary to facilitate vector replication in the host cell and, preferably, contain a selectable marker such as, but not limited to, a drug resistance marker, allowing selection of bacterial cells carrying the vector and various unique restriction endonuclease sites, allowing insertion into the vector of a promoter (i.e. prec), a ribosome binding site, a translation start signal codon (ATG) and a structural gene (e.g. a DNA sequence coding for the protein or peptide of interest).

In one embodiment, cloning or expression vehicles comprise plasmid DNA molecules able to replicate in at least one genus of bacteria and, preferably, achieve a high copy number and are stably maintained in transformed host cells. As used herein, the term "limited host range vector or vehicle" means the ability of a given vector to replicate in only one or a few genera of bacteria.

In a preferred embodiment, broad host range vehicles are employed. Broad host range cloning or expression vehicles useful in the present invention include plasmids which contain, minimally, an origin of replication which functions in two or more genera of bacteria and preferably also contains a gene or genes encoding the enzyme(s) or protein(s) required for the replication of the plasmid DNA. Examples of such broad host range vectors include IncP-1, IncP-2, IncW, IncN, IncQ and those described by Bukhari, A.I. et al., eds. (1977) Cold Spring Harbor Laboratory, Cold Spring Harbor, New York pp. 601-670.

In one embodiment of the present invention, broad host range expression vehicles are created by fusing a more limited host range cloning or expression vector with DNA molecules containing a broad host range origin of replication and replication genes. In a preferred embodiment, a broad host range origin of replication and IncQ replication genes (collectively, an "IncQ replicon" or "IncQ") are employed. The IncQ replicon is described by Geurry, P. et al., 1974, J. Bacteriol. 117: 619-630, which publication is hereby incorporated by reference herein. As previously described, a functional expression vector also contains DNA sequences necessary for the complete transcription of a desired structural gene and translation of messenger RNA (mRNA) molecules encoding the desired protein product.

As previously described and demonstrated more fully in the Examples below, the present invention is directed to the discovery that an E. coli recA promoter (Prec) can cause inducible transcription of a desired structural gene in heterologous Gram-negative bacteria. While it is shown herein that only Prec is required for the complete transcription of a desired structural gene into mRNA molecules, additional transcriptional control elements can also be included in expression vehicles containing Prec operably joined to a structural gene. Such additional transcriptional control elements (e.g. sequences) can include, without limitation, transcription termination sequences.

As previously discussed, the subsequent translation of mRNA molecules encoding the desired peptide or fragment thereof into the desired protein first requires binding of the mRNA molecules to host cell ribosomes. Such binding is effected by the presence, in the mRNA molecules, of a ribosome binding site or sequence, hereinafter referred to as an SD-sequence or SD. The SD-sequence is conventionally positioned between the promoter sequence and translational start signal codon and is, preferably positioned from about 5 to about 9 base pairs upstream (5′) to the translation start signal codon.

In one embodiment, the SD-sequence employed comprises the sequence:

```
    BglII           ↑↑↑↑↑↑↑           NcoI

5'-AGATCTGTTGTAAGGAGTCTAGACCATGG-3'

    TCTAGACAACATTCCTCAGATCTGGTACC
```

wherein the (↑) denotes nucleotides complementary to the *E. coli* ribosomal RNA and wherein the entire above sequence represents a synthetic sequence derived from several previously published SD-sequences (Scherer et al., 1980, NUC. Acids. Res. 8: 3895-3905). The above sequence is hereinafter referred to as a consensus (cons) ribosome binding site. The BglII and NcoI restriction sites enable insertion of said sequence into an expression vector in the desired orientation and also provide a means for creating a translation start codon immediately preceding the desired structural gene contained within the expression vectors.

In a preferred embodiment, the SD-sequence is contained within a sequence derived from the first about 104 nucleotides directly 5′ (upstream) to the translation start codon of the bacteriophage T7 gene 10 coding sequence. The complete about 104 nucleotide sequence comprises the promoter for the bacteriophage T7 gene 10 protein and the 5′ non-translated region of gene 10 mRNA. Said DNA or RNA equivalent sequence or fragment(s) thereof are hereinafter collectively referred to as a "G10L sequence" or "G10L sequences" and are more fully described in European Application Publication number 241,446 (published October 14, 1987) which application is hereby incorporated by reference herein.

The entire DNA sequence of the bacteriophage T7 genome has been published by Dunn and Studier (1983) and is hereby incorporated by reference hereto. Thus, the G10L sequences useful in the present invention can be obtained by isolation of the bacteriophage T7 DNA genome and separation of G10L sequences therefrom by techniques known to those skilled in the art or, alternatively, the G10L sequences of the present invention can be independently synthesized by conventional means.

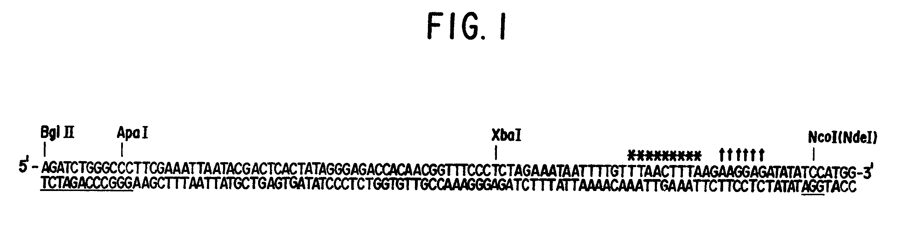

In one embodiment of the present invention, a DNA segment corresponding to the first about 104 base pairs (bp) immediately upstream of the coding sequence for the bacteriophage T7 gene 10 was constructed by chemical synthesis based upon the published sequence, Dunn and Studier (1983), of the bacteriophage T7 genome. Specifically, said G10L sequence, shown in Figure 1, was constructed to correspond to the naturally-occurring G10L sequence with the following modifications. BglII and ApaI restriction endonuclease sites were inserted at the 5′-end of the G10L sequence and a NcoI restriction endonuclease site was inserted at the 3′-end of the G10L sequence. The insertion of these restriction endonuclease sites result in the nucleotide substitutions denoted by an underline in Figure 1. The insertions of these two restriction sites at the ends of the G10L sequence is made to facilitate subsequent insertion of the G10L fragment into expression vectors or vehicles. Thus, while the BglII and NcoI restriction sites represent the preferred restriction endonuclease sites for purposes of insertion of the G10L sequences into the preferred expression vectors of the present invention, other conventional restriction endonuclease sites can be alternatively employed. Additionally, other methods known to those skilled in the art for insertion into or joining of nucleic acid molecules with, for example, any available cloning or expression vehicle or chromosomal DNA can be alternatively employed. Such other methods include, without limitation, blunt-end ligation or chemical synthesis of nucleic acid fragments comprising the G10L sequence operatively joined to a desired structural gene (Maniatis et al., 1982, Molecular Cloning: A Laboratory Manual, Cold Spring Harbor Laboratory, Cold Spring Harbor, New York).

While the complete about 104 base pair (bp) G10L sequence contains a bacteriophage T7 promoter sequence, said sequence is not recognized by *E. coli* RNA polymerase molecules and has been shown to require its own, T7 encoded, RNA polymerase for activity. Indeed, as is shown in Example 5, below, the T7 gene 10 promoter is unable to cause regulatable expression of desired structural genes in all bacteria into which it is inserted. The diminimous production of desired gene product detected in transformed organisms carrying expression vectors in which *Prec* is deleted, is believed to be due to read-through from other promoters carried on the expression vehicles.

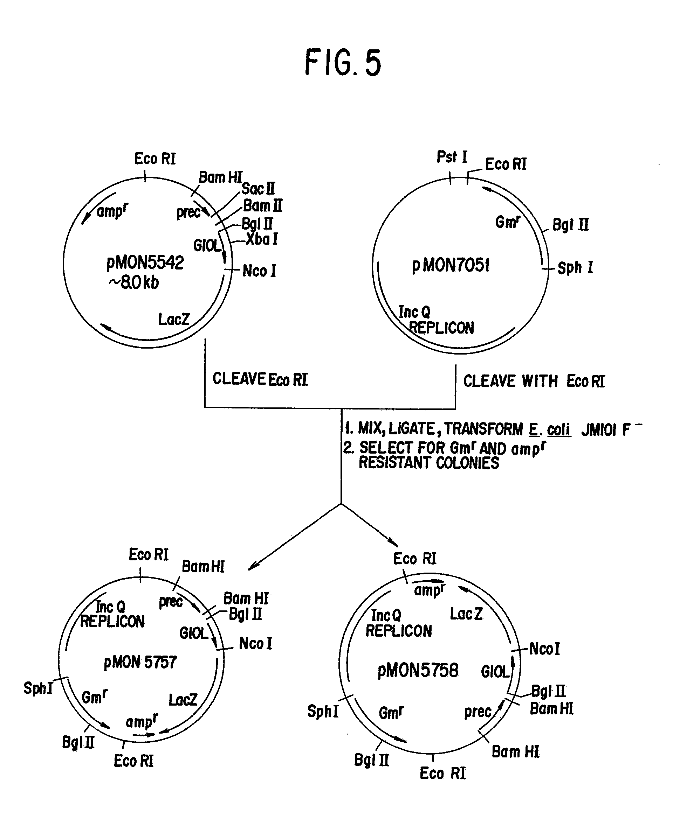

In prefered embodiments of the present invention, broad host range expression vehicles are constructed so as to comprise the following DNA sequences operatively joined sequentially as follows: *Prec*, G10L, a translation start signal codon and a structural gene. Such vectors also contain an IncQ replicon and preferably contain a selectable marker. Specific examples of such vectors include, without limitation, pMON5756, pMON5757 and pMON5758 (FIG.5), are described more fully in the Examples, below.

Once such vectors are constructed, the components can be readily exchanged for alternative components by conventional means. For example, by employing the indicated restriction endonuclease sites flanking the G10L sequence and/or structural gene, alternative SD-sequences and/or alternative structural genes can be inserted by enzymatic or chemical linkage. Additionally, alternative antibiotic resistance genes can be substituted as a transformation marker.

## 4. Host Cells

As demonstrated herein, the expression vehicles embraced by the present invention can be employed to transform such Gram-negative bacteria as *E. coli, Pseudomonas, Serratia, Erwinia, Proteus, Xanthomonas,*

*Citrobacter, Salmonella, Vibrio, Aeromonas, Zymomonas, Flavobacterium, Alcaligenes, Enterobacter, Klebsiella, Gluconobacter, Rhizobium, Agrobacterium* and *Bradyrhizobium*. The term "heterologous", when applied to Gram-negative bacteria, is herein understood to mean bacteria which do not naturally contain an *E. coli rec*A promoter. The terms "transform" and "transformation" are herein understood to comprise any method for introducing exogenous DNA into the genome of a host cell. Such methods include, without limitation, transformation, transduction, conjugation, transfection and integration into chromosomal DNA. The term "gemone" is herein understood to mean both chromosomal and extrachromosomal DNA.

The transformed host cell is then selected (Maniatis et al., 1982) and cultured under conditions which cause the expression of the desired structural gene. As demonstrated more fully in the Examples below, P*rec* effectively causes the constitutive expression of desired structural genes operatively joined thereto. Indeed, in transformed *Pseudomonas*, non-induced P*rec* expression levels of desired product are higher than in *E. coli*. Additionally, and most significantly, P*rec* is able to cause inducible expression of desired structural genes in heterologous Gram-negative bacteria.

The desired protein so produced can then be purified by conventional techniques and/or assayed for production by conventional means consistent with the protein produced. Such purification and/or protein assay methodologies can also be employed to ascertain the level(s) of protein produced.

The following microorganisms have been deposited in accordance with the Budapest Treaty with the American Type Culture Collection, 12301 Parklawn Drive, Rockville, Maryland, 20852, U.S.A.:
ATCC - 39936 *E. coli* W3110
ATCC - 53469 *E. coli* N6405
ATCC - 67043 *E. coli* M5219 (pMON5510)
ATCC - 67044 *E. coli* JM101 (pMON6002)
ATCC - 53023 *E. coli* W3110 (pMON3213)

## EXAMPLES

Materials and Methods

All oligonucleotides are synthesized employing an Applied Biosystems DNA synthesizer in accordance with the procedure set forth by the manufacturer, Applied Biosystems, Inc. (Foster City, California). $^{32}$P-labeled nucleotides are obtained from Amersham (Arlington Heights, Illinois).

Unless otherwise noted, all specialty chemicals are obtained from Sigma (St. Louis, Missouri). Restriction enzymes and DNA modifying enzymes are obtained from New England Biolabs (Beverly, Massachusetts), New England Nuclear (Boston, Massachusetts) and Bethesda Research Laboratories (Garthersburg, Maryland) and used in accordance with manufacturer's directions. T4 DNA ligase is obtained from Promega Biotec (Madison, Wisconsin) and used in accordance with manufacturer's specifications.

*E. coli* JM101, *Pseudomonas testosteroni* and *Serratia marcescens* may be obtained from the American Type Culture Collection (ATCC) (Rockville, Maryland) under ATCC accession numbers 33876, 17459 and 25419, respectively. *Pseudomonas putida* mt-2 is obtained from M. Bagdasarran (Max Plank Institute for Molecular Genetics, D-1000 Berlin-Deblin, F.R.G.) and is described by Murray, K. et al. Eur. J. Biochem. (1972) 28: 301-310. *Pseudomonas aeruginosa* 2003 is obtained from M. Vasil (Univ. Colorado Med. School, Denver, Colorado) and is described by Vasil, M. L. et al. J. Bacteroil (1982) 152: 431-440. *Pseudomonas syringae* JL2000 is obtained from J. Loper (Dept. Botany and Plant Pathology, Oregon State University, Corvallis, Oregon 97331) and is described by Loper, J. E. et al. J. Gen. Microbiol, (1984) 130: 1507-1515. *Erwinia herbicola* #26 is obtained from the collection of A. Kelman (University of Wisconsin, Madison, Wisconsin). *Pseudomonas fluorescens* 701 E1 is of the type described by D. Drahos et al. Biotechnology (1986) 4: 439-444. *Pseudomonas fluorescens* 1141F1 is a field isolate from the collection of B. Hemming (Monsanto Co., St. Louis, Missouri). *E. coli* strain W3110 can be obtained from the ATCC (Rockville, Maryland) under ATCC accession number 39936. *E. coli* strain N6405 can be obtained from Dr. M. Gottesman, National Institutes of Health (Bethesda, Maryland) and can be obtained from the ATCC under accession number 53469. *E. coli* strain BW313 can be obtained from Dr. Thomas Kunkel, Laboratory of Genetics, National Institute of Environmantal Health Sciences, Research Triangle Park, North Carolina, 27709. Vectors pBR327 and M13mp9 can be obtained from Pharmacia (Piscataway, New Jersey). Plasmid pUC18, described by Yanisch-Perron et al. (Gene (1985) 33: 103-119), can be obtained from Pharmacia (Piscataway, New Jersey). Vector M13mp19 can be obtained from New England Biolabs (Beverly, Massachusetts).

*E. coli* JM101 F⁻, which lacks the F factor beta-galactosidase complementary sequences, is created from *E. coli* JM101 to avoid homologous recombination between the plasmid and chromosomal sequences. Conversion of *E. coli* JM101 to JM101 F⁻ is achieved in accordance with the method described by Hirota, Y., Proc. Nat'l. Acad. Sci., U.S.A. (1960) 46: 57-64.

The growth media for the bacteria and conditions for transformation and selection of *E. coli* cells carrying expression vehicles containing a conventional antibiotic resistance marker are for example as essentially described in Maniatis et al. eds. (1982) *Molecular Cloning A Laboratory Manual*, Cold Spring Harbor Laboratory, Cold Spring Harbor, New York.

All bacterial growth media components and antibiotics are obtained from either Sigma (St. Louis, Missouri) or Difco Laboratories (Detroit, Michigan). Specifically, cells containing the desired plasmids are grown in the presence of 50μg/ml kanamycin to select for the presence of expression vehicles containing a kanamycin resistance (Kn$^r$) marker. Cells carrying expression vehicles containing ampicillin resistance (amp$^r$) or gentamicin resistance (gen$^r$) markers are grown in medium containing 200 μg/ml ampicillin or 10 μg/ml gentamicin, respectively. For *P. testosteroni*, 500 μg/ml gentamicin is used for selection. The growth media used is LB medium (Maniatis et al., 1982) and growth achieved by incubation of bacterial cultures at 30°C or 37°C with shaking at 250 rpm.

Rifampicin resistance derivatives of the above bacterial strains, except the *E. coli* strains, are generated to provide a counter-selectable marker for conjugable transfer of the desired vectors. A 100 μl aliquot of each strain grown overnight at 30°C on LB media is spread on the surface of a petri plate containing LB medium with 50 μg/ml rifampicin. Spontaneous rifampicin resistance mutants are selected and purified by restreaking an isolated colony on another LB plate containing rifampicin (50 μg/ml). These rifampicin-resistant derivatives are useful in triparental matings with HB101(pRK2013) and the appropriate *E. coli* culture containing the desired plasmid using the method described by Ditta, G.S. et al. (Ditta, G. S. et al. Proc. Nat'l. Acad. Sci., U.S.A., 1980, 77: 7347-7351). Selection of recombinant cells capable of producing beta-galactosidase are selected on LB medium containing rifampicin (50 μg/ml) and kanamycin (50 μg/ml), described above.

Induction of transcription from the *E. coli rec*A promoter is conducted as follows. Recombinant bacteria carrying expression vehicles are grown in LB medium (Maniatis et al., 1982) containing 50 μg/ml kanamycin to exponential phase, typically to a cell density of about 100 Klett Units (measured with a Klett-Summerson meter, Klett Mfg. Co. New York City, New York). A 5 ml sample is then removed and nalidixic acid (10 mg/ml in 0.1 N NaOH) added to the remainder of the culture to a final concentration of 50 μg/ml naladixic acid. Growth is continued for several hours after induction with aliquots taken a designated (e.g. hourly) intervals to determine the peak of desired protein production. A high level of aeration is maintained throughout the bacterial growth in order to achieve maximal production of the desired gene product and the temperature of the culture is maintained at either 30°C or 37°C.

The levels of desired polypeptide produced in recombinant host cells are determined by such protein-specific assays as enzyme activity and Western immunoblotting (Renart et al. (1979) Proc. Nat'l. Acad. Sci., U.S.A. 76: 3116-3120). For example, the levels of beta-galactosidase (β-gal) produced are determined as follows. Cells containing expression vehicles are grown in LB medium containing an antibiotic concentration appropriate for the resistance marker carried on the expression vehicle as described above, until they reached an optical density of about 100 Klett units. Cells from 5.0 ml of culture are harvested by centrifugation and the pellet is resuspended in 1.0 ml Z Buffer (comprising 60 mM monobasic sodium phosphate, 40 mM dibasic sodium phosphate, 10 mM potassium chloride, 50 mM beta-mercaptoethanol, adjusted to pH 7.0). The extract is diluted 2 to 100 fold with Z Buffer prior to assay in order to get a measurable activity. 25 to 100 μls of diluted sample is added to Z Buffer to a final volume of 1.0 ml. The tubes are equilibrated at 37°C and 0.2 ml of O-nitrophenyl-β-galactopyranoside (ONPG) (4 mg/ml in 0.1 M phosphate buffer, pH 7.0) is added. The reaction is allowed to proceed until a noticeable yellow color developed. The reaction is then stopped by adding 0.5 ml 1M $Na_2CO_3$, and the absorbance is measured at 420 nm. Beta-galactosidase activity is expressed as μmoles of product formed per minute per mg of protein using the μM extinction coefficient of 4.5 for the ONPG in accordance with the method described by Miller, J. W. (1972) Experiments in Molecular Genetics, Cold Spring Harbor Laboratory, Cold Spring Harbor, New York. Protein concentration is determined as described by Braford, M.M., Anal. Biochem. (1976) 72: 248-254.

The levels of porcine growth hormone (PGH) are determined by Western immunoblotting in accordance with the method described by Renart et al. (1979). Antibodies to PGH are made by conventional techniques.

The following plasmids are described in European Patent Application publication number 241,446 (published October 14, 1987) which application is incorporated herein by reference: pMON6002, pMON5515, pMON5514, pMON5537, pMON5510, pMON5539, pNDC8, pBGH$_{ex-1}$ and M13mp9/BGH. Said concurrently filed application and published application are hereby incorporated by reference herein.

## Example 1

This example describes the construction and assembly of the synthetic G10L molecule shown in Figure 1. The synthetic double-stranded DNA (ds DNA) G10L molecule comprises approximately the first 100 base-pair (bp) immediately 5′ to the translation start codon (ATG) of the bacteriophage T7 gene 10 coding sequence, and an ATG start codon immediately followed by a G-C bp, with bp substitutions denoted by an underline in Figure. 1.

In order to produce the ds DNA molecule shown in Figure 1, six complementary and partially overlapping synthetic oligonucleotides are synthesized as shown in Figure 5. Aliquots of the crude synthetic oligonucleotides are purified by electrophoresis on polyacrylamide-urea gels, 16% (w/v) in 7M urea (Maniatis et al., 1982). The concentration of the synthetic DNA in each preparation is determined by quantitative 5′-end labeling reactions using γ-$^{32}$P-ATP at a specific activity of 22,000-24,000 counts per minute (cpm) per mole of ATP, and T4 DNA kinase.

Assembly of synthetic DNA segments 1-6 (Figure 5) is performed as follows. All six oligonucleotides are phosphorylated at their 5′-ends with polynucleotide kinase. Complementary pairs of oligonucleotides (1/2, 3/4 and 5/6) are mixed together in pairs at a concentration of 5 picamoles/μl each, in T4 ligase buffer described by the manufacturer. Each pair is then heated to 75°C for 15 min. and the mixtures are then allowed to cool slowly (i.e. anneal). The three pairs of oligonucleotides are then mixed together and ligated overnight at 15°C. The mixture is then treated with BglII and NcoI restriction endonucleases to eliminate any polymers of the G10L that had formed and the assembled G10L fragment is purified by electrophoresis on a non-denaturing 12% (w/v) polyacrylamide gel (Maniatis et al. 1982). The resultant 104 bp DNA molecules were electroeluted from the gel to yield molecules having the DNA sequence shown in Figure 1.

## Example 2

This example demonstrates the construction of various expression vehicles comprising an *E. coli recA* promoter operatively linked to a synthetic G10L sequence and/or a structural gene. Specifically, this example demonstrates the construction of broad host range expression vehicles comprising an *E. coli recA* promoter (P*rec*) operatively linked to the following: an *E. coli* beta-galactosidase (*lacZ*) structural gene and a consensus *E. coli* ribosome binding (S.D.) sequence; an *E. coli* beta-galactosidase structural gene and a G10L sequence; a porcine growth hormone (PGH) structural gene and a G10L sequence.

### a. P*rec* - G10L-*lacZ*

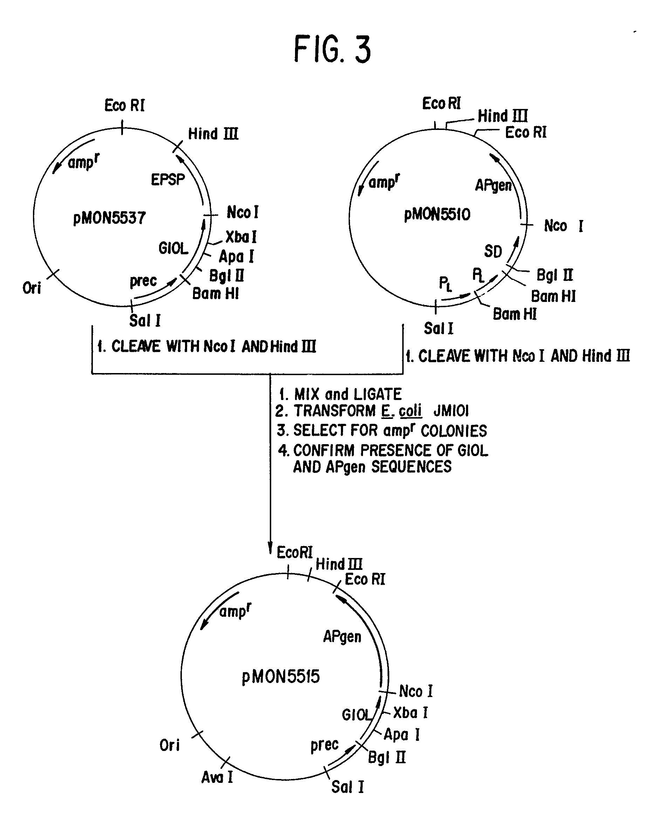

The recombinant expression vector pMON6002 comprising a pBR327 plasmid having inserted therein an *E. coli recA* promoter (P*rec*), a consensus SD-sequence (SD) and an *E. coli* 3-enolpyruvyl shikimate 5-phosphate syntase (EPSP) DNA coding sequence is digested with restriction endonucleases BglII and NcoI and the large vector fragment is purified by chromatography on NACS resin (Bethesda Research Laboratories, Gaithersburg, Maryland) in accordance with manufacturers instructions. Thereafter the vector fragment is mixed with synthetic G10L molecules (FIG. 1), in the presence of T4 DNA ligase to produce plasmid pMON5537 (FIG. 3). Plasmid pMON6002 can be obtained from the ATCC (Rockville, Maryland) under ATCC accession number 67044. The resultant pMON5537 vector is then used to transform *E. coli* JM101 cells. Transformed *E. coli* host cells are selected by growth in medium containing 200 μg/ml ampicillin. The creation of expression vector pMON5537 is verified by isolating the expression vector from transformed cells and digesting the isolated vector with restricting enzymes characteristic for the G10L and EPSP sequences.

As shown in Figure 3, the expression vector pMON5510, isolated from *E. coli* strain M5219 having ATCC accession No. 67043, which vector comprises a pBR327 plasmid having inserted therein two tandem copies of the bacteriophage lambda $P_L$ promoter($P_L$), a consensus SD-sequence (SD) and a DNA coding sequence for rat atriopeptigen (APgen), is digested with NcoI and Hind III and the Apgen coding sequence isolated by polyacrylamide gel electrophoresis (PAGE) (Maniatis et al., 1982). The Apgen coding sequence is then mixed, in the presence of T4 DNA ligase, with pMON5537 which had been previously digested with NcoI and HindIII and treated with calf intestine alkaline phosphatase (CIAP). The resultant pMON5515 vector is then used to transform *E. coli* JM101 cells and transformants selected by growth on LB agar plates containing ampicillin. Insertion of the Apgen DNA coding sequence into the pMON5515 vector is confirmed by digesting the isolated vector with restriction enzymes characteristic for the G10L and Apgen sequences.

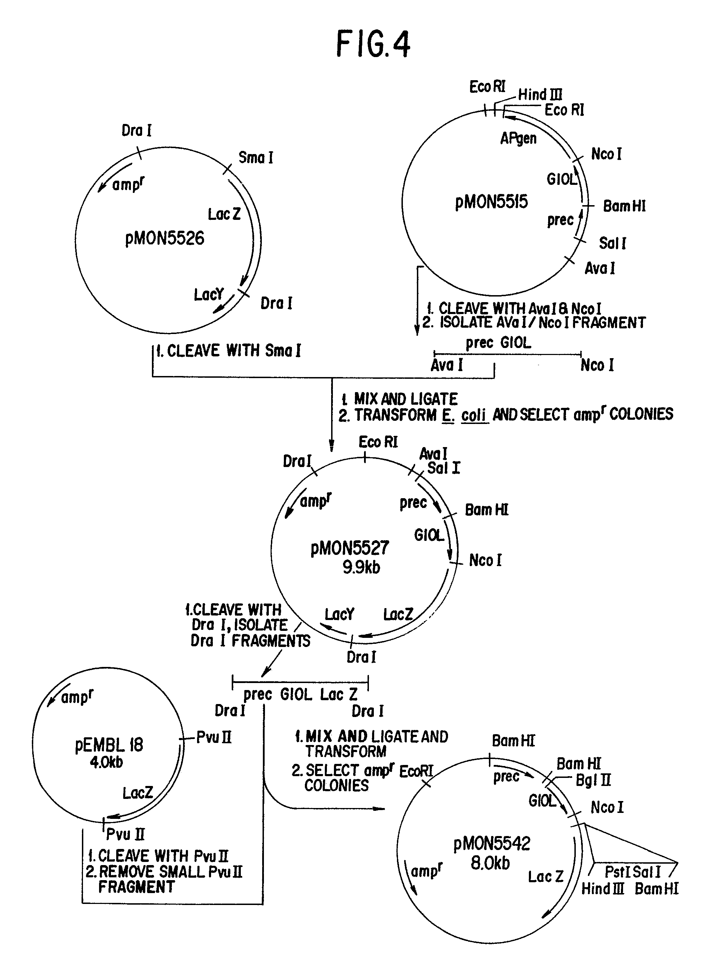

As shown in Figure 4, an expression vehicle designated pMON5542 containing an *E. coli recA* promoter (P*rec*) operatively linked to a G10L sequence and *lacZ* structural gene is created as follows. Plasmid pNM480 described by Nigel P. Minton (Gene (1984) 31: 269-273) which carries the *lacZ* structural gene is cleaved with NCOI and the NcoI site removed by filling in with *E. coli* DNA polymerase, Klenow fragment, and then religated with T4 DNA ligase to create pMON5526 (Fig. 4). Plasmid pMON5515 (FIG. 3) is cleaved with AvaI and NcoI and the small AvaI/NcoI fragment carrying P*rec* and G10L is isolated and mixed, in the presence of T4 DNA ligase with pMON5526 previously cleaved with SmaI. The ligation mixture is then used to transform *E. coli* MC1000 cells and transformants selected by growth in ampicillin-containing medium. The desired resultant vector, designated pMON5527 (FIG. 4), containing a P*rec*, G10L and *lacZ* structural gene is confirmed by restriction digestion and plating on MacConkey agar plates containing ampicillin.

Plasmid pEMBL18 described by L. Dente eds. et al. in DNA Cloning vol. 1. "A Practical Approach" pgs. 101-108, IRL Press, 1985, McLean, Virginia, is cleaved with PvuII and the small fragment removed. Plasmid pMON5527 is cleaved with DraI and an approximately 4800-5000 base pair (bp) fragment containing the P*rec*, G10L and *lacZ* sequences isolated and mixed, in the presence of T4 DNA ligase, with the PvuII cleaved pEMBL18 plasmid. The ligation mixture is then used to transform *E. coli* MC1000 and transformants selected by growth on plates comprising MacConkey agar containing ampicillin. The resultant expression vehicle carrying P*rec* operatively joined to a G10L and LacZ structural gene sequences is designated pMON5542 (FIG 4). The operative joining of said sequences is confirmed by digestion with restriction enzymes.

In order to create a broad host range expression vehicle containing the P*rec*, G10L and *lacZ* sequences, pMON5542 is joined to a broad host range plasmid pMON7051. Plasmid pMON7051, which contains an origin of replication and IncQ replication genes operable in a wide range of Gram-negative bacteria, is constructed as follows. The gentamicin resistance gene (Gm^r) is isolated from plasmid PPH1JI (Hirsch, P. R. and and Beringer, J. E., 1984, Plasmid 12: 139-141) as an EcoRI/BamHI fragment. The Gm^r-containing EcoRI/BamHI fragment is then ligated into the *E. coli* cloning plasmid pKC7 (Rogers, S.G. et al., 1979, Gene 7: 79) which had been cleaved with EcoRI and BamHI. The ligation mixture is used to transform E. coli and transformants selected by growth in medium containing both ampicillin and gentamicin. The size of the Gm$^r$ gene is then reduced to about 1900 bp by delection using SphI at the BamHI-proximal end. An EcoRI-proximal HindIII site is then inactivated by treatment of this site with Klenow polymerase and re-ligation. The EcoRI/SphI Gm$^r$-containing fragment is then cloned into an IncQ replicon, RSF1010, described by Geurry, P. et al., 1974, J. Bacteriol. 117: 619-630, to form pMON7051. Specifically, the Gm$^r$ gene fragment is first inserted into the most distal SphI site in RSF1010, thereby inactivating the streptomycin resistance gene in RSF1010. The upstream sulphanilamide resistance gene and its promoter are then removed by deleting an 800 bp PstI fragment from the IncQ replicon. Plasmid pMON5542 is then joined to pMON7051 by first cleaving both plasmids with EcoRI and then mixing the cleaved plasmids in the presence of T4 DNA ligase. The ligation mixture is then used to transform E. coli JM101F$^-$ and transformants selected by growth on medium-containing both ampicillin (200 µg/ml) and gentamicin (10 µg/ml).

The resulting broad host range plasmids containing an E. coli recA promoter operatively joined to a G10L sequence and lacZ structural gene in either orientation with respect to the gentamicin resistance gene are designated pMON5757 and pMON5758, respectively, (FIG. 5).

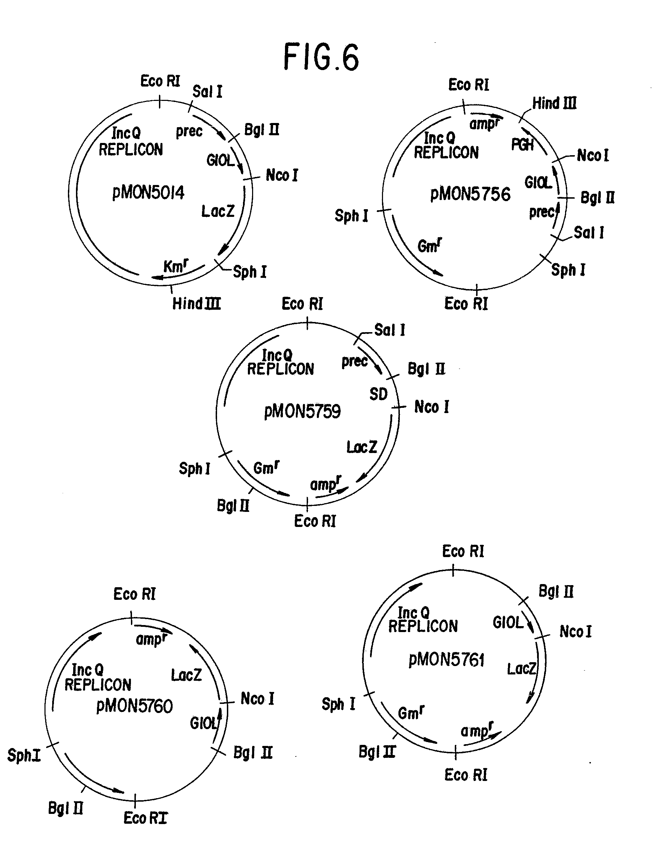

An additional broad host range expression vehicle, designated pMON5014 (FIG. 6), comprising an E. coli recA promoter operably joined to a G10L sequence and lacZ structural gene is constructed as follows.

Plasmid pMON5527 (FIG. 4) is digested with EcoRI and DraI and an approximately 4.4 kilobase (kb) fragment containing Prec, G10L and lacZ structural gene sequences isolated. This 4.4 kb fragment is then inserted into pMON7030 previously digested with EcoRI and HpaI. Plasmid pMON7030, an about 9.4 kb plasmid, comprises a pNJ9279 plasmid (Grinter, N.J., 1983, Gene 21: 133-143), obtainable from the National Collection of Industrial Bacteria, Scotland, under accession number NCIB 11715, digested with PstI to delete the Tn7 sequences while retaining the kanamycin (Km$^r$) resistance gene and broad host range replication of origin and replication genes. The resultant broad host range plasmid pMON5014 comprises a Prec operatively joined to a G10L sequence and lacZ structural gene and carries a kanamycin resistance marker to allow selection of host cells transformed with pMON5014.

### b. Prec - consensus S.D. - lacZ

Creation of a pEMBL vector comprising an E. coli recA promoter (Prec) operatively joined to an E. coli consensus ribosome binding site (consensus (SD) and lacZ structural gene, which vector is designated pMON5575, is as follows.

Plasmids pMON6002, obtained from E. coli JM101 having ATCC accession number 67044, and pMON5515, described above, are both individually digested with NcoI and EcoRI and then mixed in the presence of T4 DNA ligase. The ligation mixture is then used to transform E. coli JM101 cells and transformants selected by growth in ampicillin-containing medium. The desired vector, designated pMON5514 carrying a Prec operatively joined to a consensus S.D. and an atriol peptigen coding sequence (APgen) is confirmed by deigestion with restriction enzymes showing the absence of a G10L sequence and presence of an atrial-peptigen coding sequence. Plasmid pMON5514 is cleaved with AvaI and NcoI to obtain an about 1200 bp fragment containing Prec and consensus S.D. sequences. This fragment is then purified and inserted into pMON5526 (FIG. 4) at the SmaI site. The resulting plasmid is designated pMON5540 and comprises a pBR322 derivative plasmid containing a Prec operatively joined to a consensus S.D. and lacZ structural gene. The pMON5575 pEMBL-derivative plasmid is created by replacing the EcoRI/HindIII region of pMON5542, which carries the Prec and G10L sequences, with the EcoRI/Hind III fragment from pMON5540, which carries the Prec and consensus S.D. sequences.

The broad host range expression vehicle, designated pMON5759 (FIG. 6), is constructed by fusing pMON5575 with pMON7051. Specifically, pMON5575 and pMON7051 are individually digested with EcoRI, ligated and then used to transform E. coli JM101F$^-$ and transformants selected by growth in gentamicin- and ampicillin-containing medium. The resultant broad host range expression vehicle, pMON5759, comprises Prec operatively joined to a consensus ribosome binding site (SD) and lacZ structural gene. Vector pMON5759 also contains a G-m$^r$ gene and IncQ replicon.

### c. Prec-G10L-PGH

A broad host range expression vehicle, designated pMON5756 (FIG. 6), carrying an E. coli promoter (Prec) operatively joined to a G10L and porcine growth hormone (PGH) structural gene is created as follows.

A pBGH$_{ex-1}$ plasmid carrying a DNA coding sequence for bovine growth hormone (BGH) is obtained from Genentech, Inc., So. San Francisco, California. This plasmid can be prepared as described in European Patent Application publication No. 75,444 (published March 30, 1983); Seeburg et al., 1983, DNA 21: 37-45; Goeddel et al., 1979, Nature 281:544-548; DeBoer et al., 1982, in Promoters: Structure and Function, Chamberlin and Rodriguez, eds., chapter 293; Miozzare and Yanofsky, 1978, J. Bacteriol. 133:1457-1466; and Rosenberg and Court, 1979, Ann. Rev. Genet, 13:319-353. The pBGH$_{ex-1}$ expression vector is a pBR322 bacterial plasmid carrying a gene for BGH [BGH(P)] wherein the N-terminal amino acids of the BGH protein encoded therein are

NH$_2$-methionine (met) and phenylalanine (phe). The gene comprises, in sequence, a tryptophan promoter (p*trp*), a segment of 5′ non-translated mRNA, translation start codon immediately adjacent to the N-terminal phe (P) codon of BGH, hereinafter designated BGH(P), the BGH coding sequence and a translation termination codon.

The BGH(P) coding sequence is modified by oligonucleotide-directed site-specific mutagenesis to contain an alanine (ala) codon, immediately preceded by a N-terminal translation start (ATG) methionine codon, and NcoI restriction site overlapping the translation start (ATG) codon as follows. The BGH(P) DNA coding sequence is excised from the plasmid BGH$_{ex-1}$ as a HindIII/EcoRI fragment and cloned into the HindIII/EcoRI site of M13mp9 double-strand DNA (RF DNA). Insertion of the BGH(P) DNA coding sequence into HindIII/EcoRI restricted RF M13mp9 DNA to create recombinant vector M13mp9/BGH, is initially ascertained by colorless plaque formation on a lawn of bacteria, *E. coli* JM101 grown in 1 x YT medium employing the soft agar overlay procedure described in Maniatis et al. (1982) which included 10 ml 100mM IPTG (isopropyl-β-D-thiogalactopyranoside) and 50 μl 2% (w/v) X-GAL(5-bromo-4-chloro-3-indolyl-β-D-galacto-pyranoside) in 3 ml of top agar, and transfected with said recombinant vector as described in Maniatis et al. (1982). Insertion of the BGH(P) coding sequence is confirmed by cleavage of RF DNA isolated from colorless plaques, Maniatis et al. (1982), of the recombinant vector with HindIII and EcoRI which yields a 590 bp fragment comprising the inserted sequence. The 590 base pair (bp) fragment is identified by agarose gel electrophoresis in one percent (w/v) agarose as described in Maniatis et al. (1982). All subsequent restriction fragments are identified by this referenced method. The isolation of single-stranded (ss) phage DNA is conducted in accordance with the method of Messing et al. (1982) Gene 19: 269. The M13mp9 BGH vector is then employed as a template in the oligonucleotide-directed site-specific mutagenesis essentially as described by Zoller and Smith (1982) NUC. Acids Res. 10: 6487-6500, Zoller and Smith (1983) Methods in Enzymol. 100: 468-500, and Norris et al. (1983) NUC. Acids Res. 11: 5103-5112, the relevant portions of which are herein incorporated by reference.

The mutagenesis procedure for creation of a BGH coding sequence [BGH(A)] comprising an N-terminal met codon immediately followed by an alanine codon and NcoI site from the BGH coding sequence having an N-terminal met codon immediately followed by a phenylalanine codon and lacking an NcoI site is as follows. An oligonucleotide primer containing the sequence of the desired mutation was used to prime synthesis of a closed-circular DNA copy of the SSDNA M13mp9/BGH template. The sequence of this primer is 5′-GTGAATTCTCCATGGCTTTCCCAGCTATGTC-3′. The closed-circular dsDNA molecules thus generated are separated from incomplete and ssDNA circles by alkaline sucrose gradient centrifugation as described by Zoller and Smith (1983). The closed-circular dsDNA molecules are then used to transform *E. coli* JM101 as described by Messing et al. (1982) and the resulting colorless plaques are lifted onto nylon Biodyne® filters obtained from Pall Ultrafine Filtration Corp. (Glen Cove, New York) and screened for hybridization to a $^{32}$P-labeled form of the oligonucleotide primer used to generate the site-specific mutagenesis. The lifting of said plaques is conducted in accordance with methods described by the Pall Filter manufacturer. Hybridization screening is carried out using nylon Biodyne filters as described by Pall Ultrafine Filtration Corporation (Glenn Cove, N.Y.) in their "Protocol Guide for DNA Transfer to Pall Biodyne® A Nylon Filters" (1983). Filters are washed at increasing temperatures until the radiolabeled signal was removed from a control filter which is prepared with M13mp8/bGH$_{ex-1}$ phage. A typical filter washing protocol employed a room temperature wash in 6xSSC (0.9M NaCl and 0.09M NaCitrate) for ten minutes followed by a 50° wash in 6xSSC for five minutes and subsequent washings at temperatures increasing by 5°C. Plaques which hybridized to radiolabeled oligonucleotide primer at temperatures higher than the control phages are presumed to carry the newly created BGH(A) coding sequence and are termed potential positives. Alternatively, individual colorless plaques are picked from the *E. coli* JM101 transformations and grown in 5 milliliters (ml) of 2 x YT medium comprising (1.6% (w/v) tryptone, 1.0% (w/v) yeast extract and 0.5% (w/v) NaCl, overnight at 37°C with aeration. Phage DNA, prepared in accordance with Messing et al. (1982) is then spotted onto nitrocellulose, hybridized with radiolabeled primer, and washed in increasing temperatures as described above. Phage DNA which showed hybridization temperatures higher than M13mp9 BGH control plaques are similarly termed potential positives. Potential positive plaques from both screening procedures are grown as described above and used to prepare ss phage DNA, which was then sequenced according to the procedure of Sanger et al. (1977) Proc. Nat'l. Acad. Sci., U.S.A. 74: 5463, to confirm that they carried the BGH(A) coding sequence. The resultant recombinant M13mp9 phage DNA containing the BGH(A) coding sequence and NcoI restriction site is designated PNCD8.

Next, a pBR327 plasmid comprising a G10L sequence operatively joined to a BGH(A) coding sequence is constructed. Specifically, pNCD8 is digested with HindIII and NcoI and the 580 bp DNA fragment containing the BGH(A) coding sequence isolated. The 580 bp DNA fragment is then mixed, in the presence of T4 DNA ligase, with pMON5515 previously digested with NcoI and HindIII and treated with calf intestine alkaline phosphatase. The mixture is then employed to transform *E. coli* JM101 and transformants selected by growth in ampicillin-containing medium. The resultant recombinant plasmid comprising a pBR327 plasmid having inserted therein a P*rec* sequence, a G10L sequence and a DNA sequence coding for BGH(A) is designated pMON5539.

A DNA sequence coding for a porcine growth hormone [PGH(A)] protein (e.g. a porcine growth hormone protein containing an N-terminal alanine) is then inserted into pMON5539 in place of the BGH(A) coding sequence as follows. An NcoI restriction site is introduced at the 5′-end of the PGH(A) coding sequence carried in pMON3213 by oligonucleotide-directed site-specific mutagenesis. pMON3213 can be obtained from *E. coli* W3110 having ATCC accession number 53023 and is described in European Patent Application publication number 193,515 (published September 9, 1986) which application is hereby incorporated herein by reference. Specifically, pMON3213 is cleaved with EcoRI and HindIII and a resulting 590 bp fragment isolated and inserted into M13mp19. The resulting recombinant M13mp19 DNA is passaged twice through *E. coli* strain BW313 according to the procedure of Kunkel (1985) Proc. Nat'l. Acad. Sci., U.S.A., 82: 488-492, in order to incorporate uracil residues at a portion of the thymidine positions in the recombinant M13mp19 DNA. Single-stranded (ss) DNA forms of the recombinant M13mp19 DNA are isolated in accordance with the method of Messing et al., (1982) and employed as templates in the oligonucleotide-directed site-specific mutagenesis as described by Zoller and Smith (1982, 1983) and Norris et al., (1983). *In vitro* synthesis of the homologous DNA strand is primed with the following 27-base oligonucleotide primer, sequence:

```
     EcoRI   NcoI
                  metalaphepro
CAGTGAATTCTCCATGGCCTTCCCAGC
```

Following second (e.g. homologous) strand synthesis, the DNA is inserted into a wild-type *E. coli* strain, JM101, to enrich for plaques containing the mutated DNA.

Four clear plaques resulted from the insertion of the mutated DNA into the wild-type *E. coli* JM101. The plaques are selected and grown overnight in 2 x YT medium at 37°C. Single-stranded DNA is prepared and sequenced by the dideoxy-chain termination method of Sanger et al. (1977). Two of four DNA's sequenced contained the NcoI site. Presence of the additional restriction site is also confirmed by NcoI digestion. The PGH(A) gene with the NcoI site at the 5'-end, cloned in M13mp19, is designated pMON3267.

The PGH(A) DNA coding sequence is then isolated as an NcoI/HindIII fragment following digestion of pMON3267 with NcoI and HindIII. The PGH(A) DNA coding sequence is then mixed, in the presence of T4 DNA ligase, with pMON5539 previously digested with NcoI and HindIII. The resultant plasmid is then employed to transform *E. coli* JM101 which are grown in ampicillin-containing medium to select for transformants. The resultant plasmid contains the following DNA segments sequentially operatively linked: P*rec*, a G10L (e.g. 104 bp) sequence, and a PGH(A) coding sequence.

Plasmid pMON5756 (FIG. 6), is constructed by ligating the above plasmid previously cleaved with EcoRI, to pMON7051, previously cleaved with EcoRI, as described above. Plasmid pMON5756 is a broad host range expression vehicle comprising an IncQ replicon and P*rec* operatively joined to a G10L sequence and PGH structural gene.

### Example 3

This example demonstrates the ability of the *E. coli rec*A promoter (P*rec*) to cause expression of a desired structural gene in several heterologous Gram-negative bacteria.

As shown in Table I, below, various *Pseudomonas* species transformed with an expression vehicle containing P*rec* operably joined to the *E. coli* beta-galactosidase structural gene (*lacZ*) exhibited high levels of uninduced beta-galactosidase (β-gal) activity. *E. coli* JM101F⁻ and *Pseudomonas (P). fluorescens* 701EI and *P. testosteroni* are all individually transformed with a β-gal expression vehicle, pMON5014, described above, in accordance with the methods previously described. All transformed bacteria are grown at 30°C in LB medium containing 50 μg/ml kanamycin as previously described. Basal levels of β-gal produced in the transformed bacteria are determined as described above, and are expressed in micromoles (μM) per minute (min) per milligram (mg) of protein. Surprisingly, both recombinant *Pseudomonas* species assayed showed higher levels of β-gal production than the transformed *E. coli* host in which the *lacZ* structural gene is endogenous.

Table I

Expression of β-gal Under Non-Inducing Conditions

| Organism | Plasmid | β-gal Specific Activity (μM/min/mg) |
|---|---|---|
| *E. coli* JM101F⁻ | pMON5014 | 2.4 |
| *P. fluorscens* (701 E1) | pMON5014 | 15 |
| *P. testosteroni* (ATCC 17409) | pMON5014 | 10 |

As shown in Table 1, above, P*rec*, when operably joined to the *lacZ* structural gene, is able to cause expression of beta-galactosidase (β-gal) in both *E. coli* and various transformed *Pseudomonas (P.)* species. Indeed, both recombinant *Pseudomonas* species tested exhibited higher levels of β-gal production than the *E. coli* species transformed with the P*rec*containing expression vehicle.

Example 4

This example demonstrates the inducibility of P*rec* in heterologous Gram-negative organisms. Specifically, P*rec* is shown to be inducible by nalidixic acid in such heterologous Gram-negative bacteria as *Pseudomonas, Serratia* and *Erwinia*. The inducibility of P*rec* in Gram-negative bacteria containing expression vehicles comprising P*rec* operably linked to a desired structural gene is shown in Tables 2-4, below.

In one study, *E. coli* JM101, *P. fluorescens* 701E1 and *P. testosteroni* are transformed with expression vehicle pMON5014 (FIG. 6). The transformed (recombinant) bacteria are grown in LB medium at 30°C, unless otherwise specified, and induced with nalidixic acid (final concentration of 50 μg/ml) as previously described. β-gal activity is then measured at 4 hours post-induction and the level of induction determined by comparing the four hour level of β-gal activity with the β-gal activity level just prior to addition of nalidixic acid (0 hours). The results of this study, shown in Table 2, below, clearly demonstrate that the P*rec* is recognized and inducible in such other non-*E. coli* Gram-negative genera as *Pseudomonas*.

Table 2

Induction of β-gal Activity by Nalidixic Acid

| Organism* | Induction Time (hr) | Sp Act μM/min/mg | Fold Induction |
|---|---|---|---|
| *E. coli* JM101F⁻ (30°C) | 0 | 3.5 | - |
| | 4 | 9.9 | 2.8 |
| *E. coli* JM101F⁻ (37°C) | 0 | 3.5 | - |
| | 4 | 11.0 | 3.2 |
| *P. fluorescens* (701E1) | 0 | 16.0 | - |
| | 4 | 51.0 | 3.1 |
| *P. testosteroni* (ATCC 17409) | 0 | 9.7 | - |
| | 4 | 14.0 | 1.4 |

*All organisms contained plasmid pMON5014.

The efficient induction (e.g. regulation) of P*rec* which controls β-gal expression is confirmed by sodium dodecylsulfate-polyacrylamide gel electrophoresis (SDS-PAGE). Specifically, about 5 Klett Units of culture is separated on 9% (w/v) SDS-PAGE gels and the gels stained with silver as described by Wray, W. et al., 1981, Anal. Biochem. 118: 197-203.

Another demonstration that P*rec* causes regulatable expression of desired structural genes in a broad range of Gram-negative bacteria other than *E. coli* is shown in Table 3, below.

The requirement of P*rec* for expression of the desired structural gene (e.g. *lacZ*) is demonstrated by creating expression vehicles which are identical except that one vector lacks P*rec* sequences. Specifically, one of the expression vectors employed is pMON5757 (FIG. 6), described above. This vector also contains a G10L sequence operatively joined to P*rec*. The second vector employed is pMON5761 (FIG. 6), which is identical to pMON5757 except that the P*rec* sequences have been deleted. The wide range of applicibility of P*rec* regulated gene expression is also demonstrated herein as three additional species of *Pseudomonas* and two additional genera of Gram-negative bacteria, *Erwinia (E.)* and *Serratia (S.)*, are transformed with an expression vehicle comprising P*rec* operatively joined to a desired (e.g. *lacZ*) structural gene.

Expression plasmids pMON5757 and pMON5761 are introduced by transformation or conjugation into the organisms listed in Table 3, below, as previously described. Spontaneous rifampicin resistant derivatives of each organism are selected and the plasmids mated into the appropriate organism by selection for gentamicin and rifampicin resistance. All recombinant organisms containing the appropriate plasmids are then grown and induced with nalidixic acid as previously described, except that gentamicin is used instead of kanamycin.

Table 3

Induction of β-gal Activity in Heterologous Bacteria by Nalidixic Acid

| Organism | Plasmid* | Induction (hr) | β-galactosidase μM/min/mg | Fold Induction |
|---|---|---|---|---|
| E. coli (30°C) | pMON5757 | 0 | 11 | |
| | | 3 | 33 | 3 |
| | pMON5761 | 0 | 0.026 | |
| | | 3 | 0.016 | 0.6 |
| E. coli (37°C) | pMON5757 | 0 | 30 | |
| | | 3 | 77 | 2.6 |
| | pMON5761 | 0 | 0.96 | |
| | | 3 | 0.27 | 0.3 |
| S. marcescens | pMON5757 | 0 | 2.2 | |
| | | 3 | 3.6 | 1.6 |
| | pMON5761 | 0 | 0.08 | |
| | | 3 | 0.08 | 1 |
| E. herbicola | pMON5757 | 0 | 20 | |
| | | 3 | 67 | 3.4 |
| | pMON5761 | 0 | 0.78 | |
| | | 3 | 0.53 | 0.7 |
| P. putida | pMON5757 | 0 | 10 | |
| | | 3 | 23 | 2.3 |
| | pMON5761 | 0 | 0.53 | |
| | | 3 | 0.40 | 0.8 |
| P. aeruginosa | pMON5757 | 0 | 9.9 | |
| | | 3 | 13 | 1.3 |
| | pMON5761 | 0 | 0.80 | |
| | | 3 | 0.87 | 1.1 |
| P. syringae | pMON5757 | 0 | 11 | |
| | | 3 | 22 | 2 |
| | pMON5761 | 0 | 0.21 | |
| | | 3 | 0.19 | 0.9 |

*pMON5757 contains the recA promoter driving β-gal expression, whereas pMON5761 is the identical construct without the recA promoter.

The results shown in Table 3, above, clearly demonstrate both the ability of the E. coli recA promoter to cause expression of a structural gene operatively linked thereto and the ability of such expression to be regulated (e.g. induced) in a wide range of Gram-negative organisms. No induction and low level lacZ gene expression is seen for cultures in which Prec is deleted.

The results of experiments demonstrating regulatable expression of a structural gene encoding porcine growth hormone (PGH) in various Gram-negative bacteria when the gene is operatively joined to Prec are shown in Table 4, below. The previously described expression vector pMON5756 (FIG. 6), which comprises Prec operatively joined to a G10L sequence and PGH structural gene, is inserted into the organisms listed in Table 4, below, and the resultant recombinant organisms grown at 30°C in ampicillin-containing LB medium. The cultures are induced with 50 μg/ml nalidixic acid, as previously described and assayed at times 0 hours and 3 hours post-induction for PGH production by Western immunoblotting of a 15% (w/v) SDS-PAGE gel and thereafter scanned for total protein with an LKB Ultroscan XL Laser densitometer (LKB, Uppsala, Sweden) in accordance with manufacturer's instructions.

Table 4

Nalidixic Acid Induced Expression of PGH

| Organism* | Induction time (hrs) | Level of Induction** |
|---|---|---|
| E. coli JM101F⁻ | 3 | + + + + + |
| P. testosteroni | 3 | + + + + |
| P. fluorescens | 3 | + + + |
| P. putida | 3 | + + |
| S. marcescens | 3 | + + + |

* All organisms are transformed with expression vehicle pMON5756
** Levels of PGH production are ascertained by Western immunoblotting and scanning with an LKB Ultroscan XL Laser densitometer (LKB, Uppsala, Sweden).

As can be seen by the results presented in Table 4, above, three different species of *Pseudomonas* and an additional genus of Gram-negative bacteria all show inducible expression of PGH.

Example 5

This example demonstrates the inducibility of P*rec* in heterologous Gram-negative bacteria and further demonstrates that desired protein production in such heterologous bacteria can be enhanced by the operative joining of a G10L sequence to P*rec* and a desired structural gene.

*E. coli* JM101F⁻ is transformed with the expression vectors indicated in Table 5B, below, and transformants selected as described above, except that 10 µg/ml of gentamicin is used for selection. The desired expression vectors (see Tables 5A and 5B) are conjugated (e.g. transduced) into the *Pseudomonas* species listed in Tables 5A and 5B, below, using 50 µg/ml gentamicin and rifampicin for *P. fluorescens* strain 701E1 and *P. fluorescens* strain 1141F1, and 250 µg/ml gentamicin and rifampicin for *P. testosteroni*. All transformed organisms are grown and induced with nalidixic acid as previously described. Plasmids pMON5760 and pMON5761 (FIG. 6) are constructed by deleting a specific number of nucleotides so as to specifically remove P*rec*.

Table 5A

Induction of β-gal Activity by Nalidixic Acid and

Enhancement of β-gal Production by G10L Sequences

| Plasmid | Induction time (hr) | P. flourescens 701E1 | | | P. flourescens 1141F1 | | |
|---|---|---|---|---|---|---|---|
| | | β-gal* | Ind.** | Enh.*** | β-gal | Ind. | Enh. |
| pMON5757† (Prec G10L) | 0 4 | 21.0 76.0 | − 3.6 | − 14.3 | 11.0 33.0 | − 3.0 | − 20.6 |
| pMON5758 (Prec G10L) | 0 4 | 27.0 87.0 | − 3.2 | − 16.4 | 18.0 37.0 | − 2.1 | − 23.1 |
| pMON5759† (Prec cons) | 0 4 | 0.31 5.3 | − 17.0 | − − | 0.29 1.6 | − 5.7 | − − |
| pMON5760 (    G10L) | 0 4 | 1.6 1.6 | − 1.0 | − − | 2.8 1.5 | − 0.5 | − − |
| pMON5761† (    G10L) | 0 4 | 1.0 0.64 | − 0.6 | − − | 0.69 0.40 | − 0.6 | − − |

† denotes plasmids which have the lacZ gene in the same orientation.

* β-gal specific activity expressed in μM/min/mg protein.

** denotes fold induction in β-gal specific activity at 4 hours post-addition of nalidixic acid.

*** denotes fold enhancement in β-gal protein activity for plasmids containing a G10L sequence (G10L) as compared to a consensus S.D. (cons) sequence.

## Table 5B

### Induction of β-gal Activity by Nalidixic Acid and Enhancement of β-gal Production by G10L Sequences

| Plasmid | Induction time (hr) | P. testosteroni 701E1 | | | E. coli JM101F⁻ | | |
|---|---|---|---|---|---|---|---|
| | | β-gal* | Ind.** | Enh.*** | β-gal | Ind. | Enh. |
| pMON5757† (prec G10L) | 0 | 8.9 | – | – | 25.0 | – | – |
| | 4 | 18.0 | 2.0 | 19.0 | 42.0 | 1.7 | 25.0 |
| pMON5758 (prec G10L) | 0 | 7.0 | – | – | 31.0 | – | – |
| | 4 | 20.0 | 2.9 | 21.1 | 40.0 | 1.3 | 23.5 |
| pMON5759† (prec cons) | 0 | 0.33 | – | – | 0.9 | – | – |
| | 4 | 0.95 | 2.9 | – | 1.7 | 1.9 | – |
| pMON5760 ( G10L) | 0 | 0.61 | – | – | 0.83 | – | – |
| | 4 | 0.46 | 0.8 | – | 0.41 | 0.5 | – |
| pMON5761† ( G10L) | 0 | 0.56 | – | – | 0.091 | – | – |
| | 4 | 1.1 | 2 | – | 0.037 | 0.4 | – |

† denotes plasmids which have the lacZ gene in the same orientation.

* β-gal specific activity expressed in µM/min/mg protein.

** denotes fold induction in β-gal specific activity at 4 hours post-addition of nalidixic acid.

*** denotes fold enhancement in β-gal protein activity for plasmids containing a G10L sequence (G10L) as compared to a consensus S.D. (cons) sequence.

Induction of β-gal production observed via enzyme analysis in Tables 5A and 5B, above, is confirmed for both *E. coli* and *P. fluorescens* 701E1 by protein analysis on 9% (w/v) SDS-PAGE in accordance with methods previously described.

As shown in Tables 5A and 5B, above, nalidixic acid induced expression of the *lacZ* structural gene, contained within the designated expression vehicles, is due solely to P*rec*. No induction and reduced *lacZ* gene expression is observed in cultures in which P*rec* is deleted Expression is low and non-inducible in bacteria containing pMON5760 and pMON5761, both of which lack P*rec* but retain a G10L translation enhancer sequence.

As shown in Tables 5A and 5B, above, nalidixic acid induction of P*rec* is observed when either the G10L ribosome binding site is operably joined to P*rec* (pMON5757 and pMON5758) or the *E. coli* consensus ribosome binding site (pMON5759) is operably joined to P*rec*. Additionally, all transformed organisms show a 14 to 25 fold higher level of β-gal production with the vectors pMON5757 and pMON5758 which contain a G10L sequence compared to the *E. coli* consensus (cons) ribosome binding site.

The foregoing examples illustrate preferred embodiments of the present invention and are not intended to limit the invention's scope in any way. While this invention has been described in relation to its preferred embodiments, various modifications thereof will be apparent to one skilled in the art from reading this application.

**Claims**

1. A method for causing regulatable expression of a structural gene encoding a desired polypeptide in non-*E. coli* Gram-negative bacteria, the method comprising inducing an *E. coli recA* promoter operatively joined to the structural gene and thereby obtaining the polypeptide.

2. The method of Claim 1 wherein the structural gene comprises a DNA sequence encoding an eucaryotic protein.

3. The method of Claim 2 wherein the eucaryotic protein is porcine growth hormone.

4. The method of Claim 1 wherein the *E. coli recA* promoter is induced by a condition which causes damage to or modifies the bacterial DNA.

5. The method of Claim 4 wherein the condition is addition of a substance that inhibits DNA gyrase.

6. The method of Claim 5 wherein the substance is nalidixic acid.

7. The method of Claim 1 wherein the non-*E. coli* Gram-negative bacteria is selected from a group consisting of *Pseudomonas*, *Erwinia* and *Serratia*.

8. The method of Claim 1 wherein the non-*E. coli* Gram-negative bacteria is selected from a group consisting of *Pseudomonas testosteroni*, *Pseudomonas syringae*, *Pseudomonas aeruginosa*, *Pseudomonas putida*, *Serratia marcescens* and *Erwinia herbicola*.

9. The method of Claim 1 wherein the *E. coli recA* promoter operatively joined to the structural gene is carried on a plasmid.

10. The method of Claim 9 wherein the plasmid is a broad host range expression vector.

11. The method of Claim 10 wherein the broad host range expression vector comprises an IncQ replicon.

12. The method of Claim 11 wherein the vector·is selected from a group consisting of pMON5756, pMON5757, pMON5759 and pMON5758.

13. The method of Claim 1 wherein the *E. coli recA* promoter is additionally operatively joined to a G10L sequence.

14. A method for causing inducible expression of a structural gene encoding a desired polypeptide in bacteria selected from the group consisting of *Erwinia*, *Serratia* and *Pseudomonas*, the method comprising inducing an *E. coli recA* promoter operatively joined to the structural gene and thereby obtaining the polypeptide.

15. The method of Claim 14 wherein the *E. coli recA* promoter is additionally operatively joined to a G10L sequence.

16. The method of Claim 15 wherein *E. coli recA* promoter operatively joined to the G10L sequence and the structural gene are carried on a plasmid.

17. A synthetic DNA molecule comprising an *E. coli recA* promoter and a broad host range replicon.

18. The synthetic DNA molecule of Claim 17 further comprising a structural gene operatively joined to the *E. coli recA* promoter.

19. The synthetic DNA molecule of Claim 17 or 18 further comprising a G10L sequence.

20. The synthetic DNA molecule of Claim 18 wherein the structural gene comprises a DNA sequence encoding an eucaryotic protein.

21. The synthetic DNA molecule of Claim 20 wherein the eucaryotic protein is porcine growth hormone.

22. The synthetic DNA molecule of Claim 17 further comprising a selectable marker.

23. A method for producing porcine growth hormone in a non-*E. coli* Gram-negative bacteria, the method comprising inducing an *E. coli recA* promoter operatively joined to a G10L sequence and a DNA sequence encoding growth hormone and thereby obtaining growth hormone.

24. The method of Claim 23 wherein the growth hormone is selected from a group consisting of bovine and porcine growth hormone.

25. The method of Claim 23 wherein the non-*E. coli* Gram-negative bacteria is selected from the group consisting of *Pseudomonas*, *Serratia* and *Erwinia*.

26. A recombinant non-*E. coli* Gram-negative bacteria transformed with the synthetic DNA molecule of Claim 18.

27. A recombinant non-*E. coli* Gram-negative bacteria transformed with a DNA molecule comprising a broad host range replicon and an *E. coli recA* promoter operatively joined to a G10L sequence and structural gene.

28. The recombinant bacteria of Claim 27 selected from the group consisting of *Pseudomonas*, *Serratia* and *Erwinia*.

29. A method for producing a polypeptide in a non-*E. coli* Gram-negative bacteria which method comprises causing expression of a structural gene encoding the polypeptide and wherein the structural gene is operatively joined to an *E. coli recA* promoter and thereby obtaining the polypeptide.

30. The method of Claim 29 wherein the structural gene is additionally operatively joined to a G10l sequence.

31. The method of Claim 29 or 30 wherein the polypeptide comprises an eucaryotic protein.

32. The method of Claim 29 wherein the *E. coli recA* promoter operatively joined to the structural gene is carried on a broad host range expression vector.

33. A recombinant non-*E. coli* Gram-negative bacteria, the genome of which comprises an *E. coli rec*A promoter.

34. The recombinant bacteria of Claim 33, the gemone of which further comprises a G10L sequence operatively joined to the *E. coli rec*A promoter.

35. The recombinant bacteria of Claim 33 or 34 selected from the group consisting of *Pseudomonas, Erwinia* and *Serratia.*

# FIG. 1

EP 0 335 854 A2

# FIG. 2

SEGMENT # 1

5' - GATCTGGGCCCTTCGAAATTAATACGACTCACTATA-3'

||||||||||||||||||||||||||||||||||||

3' - ACCCGGGAAGCTTTAATTATGCTGAGTGATATCCCTCT -5'

SEGMENT # 2

SEGMENT #3

5' - GGGAGACCACAACGGTTTCCCTCTAGAAAT-3'

|||||||||||||||||||||||||||

3' - GGTGTTGCCAAAGGGAGATCTTTATTAAAACAAAT- 5'

SEGMENT #4

SEGMENT #5

5' - TGTTTAACTTTAAGAAGGAGATATATC -3'

|||||||||||||||||||||||

3' -TGAAATTCTTCCTCTATATAGGTAC- 5'

SEGMENT #6

Bgl II

Apa I

Xba I

Nco I

1 →

3 →

5 →

← 2

← 4

← 6

# FIG. 3

EP 0 335 854 A2

# FIG.4

# FIG. 5

# FIG.6